# DEMANDE DE BREVET EUROPEEN

(11) **EP 2 105 132 A1**
(43) Date de publication de la demande: **30.09.2009**
(21) Numéro de dépôt: 09163685.2
(22) Date de dépôt: 06.04.2007
(51) Int. Cl.: A61K 31/196, A61P 9/10

(54) **Utilisation d'un composé anti-athérothrombotique pour l'obtention de médicaments destinés au traitement des troubles vasculaires**

(30) Priorité: 07.04.2006 FR 0603082
(62) Demande divisionnaire de: 07290421.2
(71) Demandeur: Les Laboratoires Servier, 92415 Courbevoie Cedex (FR)
(72) Inventeur: Fratacci, Marie-Dominique, 78390, Bois d'Arcy (FR); De Cordoue, Agnès, 75016, Paris (FR); Lerond, Laurence, 78160, Marly-Le-Roi (FR); Verbeuren, Tony, 78540, Vernouillet (FR); Rupin, Alain, 37510, Savonnières (FR); Lavielle, Gilbert, 78170, La Celle Saint Cloud (FR)
(74) Mandataire: Dossmann, Gérard

(57) **Abrégé**

La présente invention concerne l'utilisation d'un antagoniste spécifique des récepteurs TP pour l'obtention de médicaments destinés au traitement des troubles vasculaires chez le patient présentant un antécédent d'accident vasculaire cérébral, notamment pour la réduction des événements cérébro-vasculaires et cardio-vasculaires chez des patients présentant un antécédent d'accident ischémique cérébral (AIC) ou d'attaque ischémique transitoire (AIT).

## Description

La présente invention concerne l'utilisation d'un antagoniste spécifique des récepteurs thromboxane - prostaglandines (TP) pour l'obtention de médicaments destinés au traitement des troubles vasculaires chez le patient présentant un antécédent d'accident vasculaire cérébral (AVC), notamment pour la réduction des événements cérébro-vasculaires et cardio-vasculaires chez des patients présentant un antécédent d'accident ischémique cérébral (AIC) ou d'attaque ischémique transitoire (AIT),

Le thromboxane A₂ (TXA₂) est un métabolite instable de l'acide arachidonique qui est impliqué dans la pathogenèse de nombreuses maladies cardiovasculaires.

Le TXA₂ et d'autres métabolites de l'acide arachidonique tels que les endoperoxydes (PGG₂-PGH₂), les HETE et les isoprostanes sont des ligands de récepteurs communs nommés récepteurs TP (thromboxane - prostaglandines - endoperoxydes). Leurs liaisons aux récepteurs TP entraîne des effets délétères: activation et agrégation plaquettaire, dysfonction endothéliale, vasoconstriction, et prolifération cellulaire.

L'athérosclérose est une maladie inflammatoire chronique, l'agent d'agression entraînant la réaction inflammatoire étant le cholestérol LDL qui s'accumule sous l'endothélium sous forme oxydée. L'inflammation intervient à plusieurs niveaux du processus athéromateux :
1) activation de l'endothélium et recrutement mono-lymphocytaire 2) production locale et systémique de cytokines pro-inflammatoires; 3) production de protéases de la matrice extracellulaire (métalloprotéases) entraînant la dégradation des protéines de la chape fibreuse et déstabilisation de la plaque ; 4) induction de l'apoptose des cellules de la plaque et formation d'un noyau lipidique pro-coagulant.

La plaque athéromateuse ainsi formée peut se rompre suite à des stimuli d'inflammation locale et de stress oxydatif, initiant l'agrégation de plaquettes à la surface aboutissant à l'occlusion. La survenue de thrombose sur plaque est dénommée « athérothrombose».
La rupture complique principalement (mais non exclusivement) les plaques qui présentent un noyau lipidique large occupant plus de 40% du volume total de la plaque, et une chape fibreuse fine riche en macrophages et pauvre en cellules musculaires lisses. Elle est le résultat du déséquilibre entre les contraintes hémodynamiques auxquelles la chape fibreuse est soumise et la solidité intrinsèque qui détermine sa résistance à la fracture.

Dans les stratégies thérapeutiques nouvelles de l'athérosclérose, il apparaît important de cibler l'inflammation dans la plaque. La stabilisation de la plaque athéromateuse nécessite la réduction de sa composante inflammatoire au bénéfice de la composante fibreuse.

Récemment, de nombreux travaux de recherche ont été effectués dans le but de prévenir les phénomènes liés à la production excessive de thromboxane A₂ dans les systèmes cardiovasculaire et neurovasculaire. Parmi ces antagonistes, ceux décrits dans le brevet EP 648 741 se sont avérés de puissants et sélectifs antagonistes des récepteurs TP, actifs par voie orale et ayant une longue durée d'action.

Plus particulièrement, l'acide 3-{6-[(4-chlorophénylsulfonyl)amino]-2-méthyl-5,6,7,8-tétrahydronapht-1-yl}propionique de formule (1) : 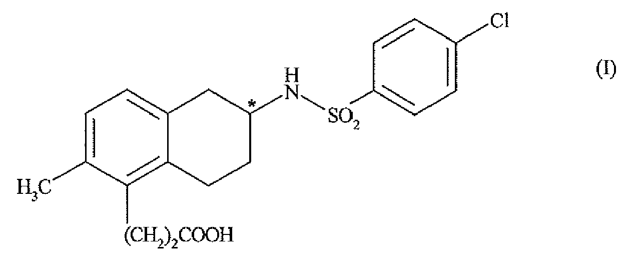 sous forme racémique ou d'isomère optiquement pur ainsi que ses sels d'addition pharmaceutiquement acceptables, est un antagoniste spécifique des récepteurs TP, qui s'est avéré avoir un puissant effet anti-athérothrombotique.

Le composé de formule (1) agit en bloquant l'agrégation plaquettaire induite par le thromboxane A₂ et les autres ligands des récepteurs TP, quelle que soit leur origine, plaquettaire ou extraplaquettaire. Il agit de plus en inhibant la vasoconstriction induite par le thromboxane A₂ et en s'opposant au dysfonctionnement endothélial et à la prolifération ainsi qu'à l'inflammation de la paroi vasculaire.

Le composé de formule (I) présente donc des propriétés : antiagrégantes plaquettaires et anti-thrombotiques, anti-inflammatoires, anti-prolifératives et anti-vasoconstrictrices.

Son effet bénéfique dans l'athérosclérose peut se traduire par l'inhibition de l'infiltration monocytaire/macrophagique dans la plaque, vraisemblablement via l'inhibition de l'expression des molécules d'adhésion vasculaires. Puisque ces cellules inflammatoires conditionnent la stabilité de la plaque, la réduction du recrutement monocytaire dans la paroi vasculaire peut, à long terme, stabiliser la plaque. En outre, l'effet anti-thrombotique du composé peut réduire l'inflammation vasculaire et l'activation endothéliale déterminées par l'adhérence des plaquettes à l'endothélium et l'interaction, de nature inflammatoire, pro-coagulante et pro-oxydante entre plaquettes, endothélium, cellules musculaires lisses et monocytes.

L'acide 3-{6-[(4-ehlorophényisulfonyl)aminoj-2-méthyl-5,6,7,8-tétrahydronapht-1-yl} propionique, ainsi que ses sels et ses isomères optiques, leurs préparations et leur utilisation en thérapeutique ont été décrits dans le brevet européen EP 0 648 741.

La demanderesse a présentement découvert que, de façon surprenante, le composé de formule (I) sous forme d'isomère actif et sous forme de sel, préférentiellement de configuration (R) et sous forme de sel de sodium possédait des propriétés intéressantes permettant de l'utiliser dans le traitement des troubles vasculaires chez le patient présentant un antécédent d'accident vasculaire cérébral, notamment pour la réduction des événements cérébro-vasculaires et cardio-vasculaires chez des patients présentant un antécédent d'AIC ou d'AIT.

De façon surprenante, le composé de formule (I) de configuration (R) préférentiellement sous forme de sel de sodium ne se comporte pas comme les autres anti-aggrégants plaquettaires : en effet, a doses faibles, l'aspirine n'a pas d'effet anti-athéroscléreux ou anti-athérogénique direct. Elle n'inhibe pas la formation de la néointima et l'accumulation de cellules inflammatoires dans la paroi vasculaire. Les effets vasculaires de clopidogrel ne sont pas non plus revendiqués chez l'Homme. Les récepteurs P2Y12, cible de clopidogrel, ne sont pas présents dans les cellules endothéliales.

Nous avons, de façon étonnante, pu mettre en évidence ces propriétés de protection secondaire du composé de formule (I) dans un modèle animale : le rat SHR-SP hypersodique. De façon surprenante, ces propriétés semblent être liées à une diminution du stress oxydant par le composé. L'importance du stress oxydant dans la pathologie cérébrale a été mise en évidence dans plusieurs publications (Margaill I, Plotkine M, Lerouet D, Free Radical Biology & Medicine, 2005, 39, 429-443 ; Crack PJ, Taylor JM, Free Radical Biology & Medicine, 2005, 38, 1433-1444). La découverte d'un impact du composé de formule (I) sur le stress oxydant dans le cadre de la pathologie vasculaire cérébrale suggère donc son utilisation possible dans une étude comme celle décrite dans la présente invention.

Les rats SHR-SP représentent un modèle expérimental bien caractérisé d'hypertension essentielle. Ces rats développent une hypertension spontanée sévère conduisant rapidement à la formation de lésions cérébrales. Ils sont très sensibles à un régime enrichi en sel ce qui induit le développement de néphroscléroses malignes (Griffin KA, Churchill PC, Picken , Webb RC, Kurtz TW, Bidani AK, Am. J. Hypertens., 2001, 14(4 Pt 1): 311-20), conduit à une augmentation de pression artérielle et de stress oxydant (Park JB, Touyz RM, Chen X, Schiffrin EL, Am. J. Hypertens., 2002, 15(1 Pt1): 78-84 ; Manning RD, Meng S, Tien N, Acta Physiol. Scand., 2003, 179:243-250) et aggrave les lésions vasculaires et l'hypertrophie cardiaque (Kyselovic J, Morel N, Wibo M, Godfraind T, J. Hypertens., 1998, 16(10): 1515-22). De plus les rats SHR-SP constituent le seul modèle animal développant spontanément des atteintes cérébro-vasculaires complexes (Volpe M, Russo R, Veccione C, Savoia C, Piras O, Gigante B, Lindpaintner K, Rubattu S, J. Hypertens., 1998, 16: S31-S35 ; Guerrini U, Sironi L, Tremoli E, Cimino M, Pollo B, Calvio AM, Paoletti R, Asdente M, Stroke, 2002, 33(3): 825-30). L'analyse cérébrale de rats SHR-SP en spectroscopie ESR (electron spin resonance) révèle un niveau élevé de stress oxydant comparé à des rats normotendus WKY (Miyazaki H, Shoji H, Lee MC, Redox Rep., 2002, 260-2657). Il a été suggéré que cette augmentation de stress oxydant entraînerait des atteintes de la barrière vasculaire cérébrale (Kim-Mitsuyama S, Yamamoto E, Tanaka T, Zhan Y, Izumi Y, Izumiya Y, Ioroi T, Wanibuchi H, Iwao H, Stroke, 2005, 36: 1077-1082) suite à des lésions des membranes lipidiques cellulaires par péroxidation et l'altération de l'ADN et des protéines. Ces atteintes cérébrales sont souvent associées à des signes cliniques comportementaux (léthargie ou agressivité, convulsions, paralysie des membres) avant la survenue d'accidents vasculaires cérébraux conduisant à la mort des animaux.

Le modèle de rat SHR-SP hypersodé apparaît comme une bonne cible pour l'évaluation du composé de formule (I) dans la prévention de l'accident cérébrale aigu. En effet, en sélectionnant la période de traitement entre 10 et 15 semaines, un modèle parfait d'épisodes d'atteinte cérébrale récurrents est obtenu. Les rats ont déjà, avant les 10 semaines, subits des attaques cérébrales non-fatals (eg des léthargies, des convulsions) et nous pouvons donc analyser si oui ou non, le composé de formule (I) est capable de prévenir ou de retarder les accidents cérébraux secondaires,

Dans ce respect, ce modèle de rat ressemble à la situation humaine et plus spécifiquement à la situation de l'étude décrite dans le brevet dans laquelle les patients ont subi un premier accident cérébral et dans laquelle l'effet d'un traitement du composé de formule (I) sur la prévention d'un second événement (cérébral ou cardiovasculaire), qui risque d'être fatal, sera étudié.

L'invention concerne donc l'utilisation du composé de formule (I) sous forme d'isomère actif et préférentiellement sous forme de sel, pour l'obtention de compositions pharmaceutiques destinées au traitement des troubles vasculaires chez le patient présentant un antécédent d'accident vasculaire cérébral, notamment pour la réduction des événements cérébro-vasculaires et cardio-vasculaires chez des patients présentant un antécédent d'AIC ou d'AIT.

Parmi les sels d'addition du composé de formule (I), on peut citer à titre non limitatif les sels d'addition à une base pharmaceutiquement acceptable comme les sels de sodium, de potassium, de *tert*butylamine, de diéthylamine, etc...

A titre préférentiel, le sel utilisé sera le sel de sodium.

Dans les compositions selon l'invention, le composé de formule (I) possède préférentiellement la configuration absolue (R).

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

La posologie utile ne varie pas selon le sexe, l'âge et le poids du patient, la nature de l'affection et des traitements éventuellement associés.

Les études ont été réalisées avec le sel de sodium de l'isomère (R) du composé de formule(I).

Dans les compositions selon l'invention les quantités de principe actif sont comprises entre 5 et 100 mg pour le sel de sodium de l'isomère (R) du composé de formule (I). De manière préférentielle, la dose journalière du sel de sodium de l'isomère (R) du composé de formule (I), sera de 30 mg par jour.

### Données pré-cliniques

L'effet anti-thrombotique du sel de sodium de l'isomère (R) du composé de formule (I), a été démontré dans différents modèles de thrombose carotidienne in vivo : électrolyse vasculaire (chien), lésion mécanique (cobaye) et stimulation électrique (rat) et ex vivo : shunt artériel carotido-earotidien par capillaire en verre: le sel de sodium de l'isomère (R) du composé de formule(I) allonge le temps d'occlusion de façon dose dépendante.
Les effets anti-prolifératif, anti-athéromateux et anti-inflammatoire du sel de sodium de l'isomère (R) du composé de formule(I) ont été explorés sur des modèles expérimentaux : il réduit significativement la surface de la lésion athérosclérotique, l'épaisseur de la paroi vasculaire et le ratio intima/media sans réduction du cholestérol sérique. Il inhibe aussi l'infiltration des macrophages dans la paroi vasculaire et l'expression des molécules d'adhésion et particulièrement des marqueurs comme l'endothéline, ICAM, VCAM.

### Modèle animale: rat SHR-SP hypersodique

### Matériels et Méthodes

### Suivi des animaux

Des rats males SHR-SP de 10 semaines d'age (Charles River) recevant 1% de NaCl dans l'eau de boisson ont été séparés en deux groupes de manière aléatoire et ont été traités ou non avec le sel de sodium de l'isomère (R) du composé de formule (1) (30 mg/kg/j) incorporé dans la nourriture.

Dans une première étude la survie de chaque groupe a été évaluée en calculant le pourcentage d'animaux vivants chaque jour après le début du traitement.

Dans une deuxième étude les rats ont été suivis pendant 5 semaines, entre 10 et 15 semaines d'age, puis ont été euthanasiés afin de quantifier un marqueur urinaire de stress oxydant (8 OH-dG), un marqueur plasmatique reflétant une inflammation aiguë (thiostatine équivalent chez le rat de la CRP) et d'évaluer l'hypertrophie cardiaque.

### Paramètres physiologiques

Durant les dernières 24 heures de suivi les animaux ont été placés en cages métaboliques individuelles et les urines ont été recueillies dans des tubes stériles (200µl EDTA 2mM). La diurèse a été mesurée et la production de 8-hydroxy-2'-deoxyguanosine (8 OH-dG) a été quantifiée par dosage ELISA (Oxis, Bioxytech).

Les rats ont été anesthésiés par injection intra-péritonéale de pentobarbital sodique (50 mg/kg, Ceva laboratories). Du sang à été prélevé au niveau de l'artère carotide (prélèvement sur héparine 5000 UI/ml) afin de mesurer la production de thiostatine par dosage ELISA (Life Diagnostics). Le coeur à été excisé, séché et pesé et l'hypertrophie cardiaque a été évaluée en divisant le poids du coeur sec par le poids corporel pour chaque animal afin de normaliser cette mesure.

### Statistiques

Les résultats sont rapportés en moyenne ± S.E.M. Les différences sont considérées significatives quand P<0.05 après un test t de Student. La survie des animaux est représentée par le graphique de Kaplan-Meier et les résultats sont considérés significatifs quand P<0.05 après un test Log-rank.

### Résultats

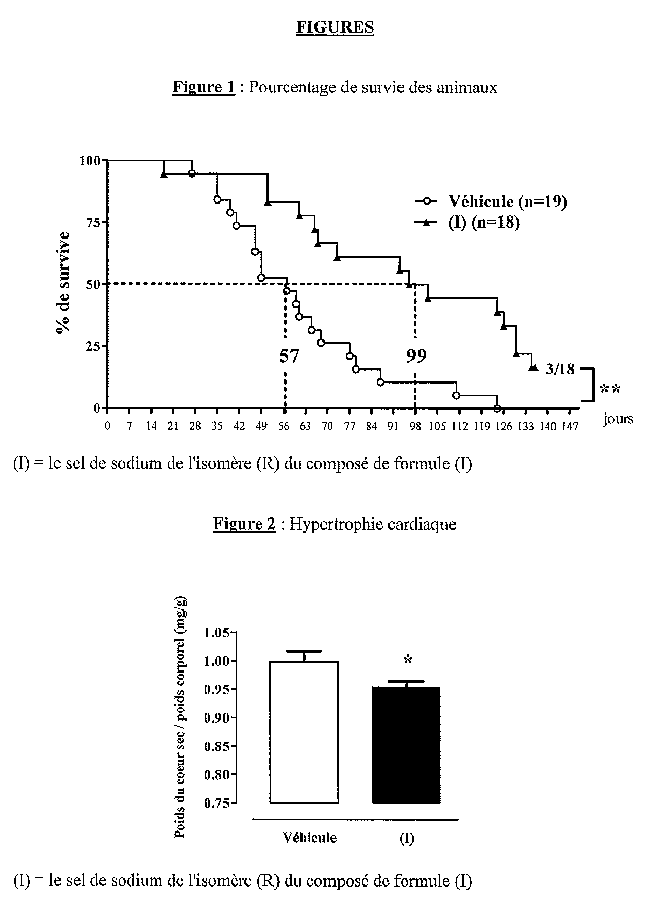

### 1) Pourcentage de survie des animaux (Figure 1)

Le pourcentage de survie des animaux a été calculé pour les deux groupes de rats. Pour le groupe Vehicule la moitié des rats sont morts 57 jours après le début du suivi alors que dans le groupe de rats traités avec le sel de sodium de l'isomère (R) du composé de formule (1) la moitié des rats sont morts après 99 jours de traitement.

Ce résultat montre que le sel de sodium de l'isomère (R) du composé de formule (I) permet de prévenir la mort des animaux donc d'améliorer le pourcentage de survie.

### 2) Hypertrophie cardiaque (Figure 2)

Les rats du groupe Vehicule ont une augmentation importante de leur masse cardiaque ce qui reflète une augmentation de la taille de cet organe ou hypertrophie. Lorsque les animaux sont traités avec le sel de sodium de l'isomère (R) du composé de formule (I) l'hypertrophie du coeur est moins importante.

Ce résultat montre que le sel de sodium de l'isomère (R) du composé de formule (I) permet de ralentir l'hypertrophie cardiaque.

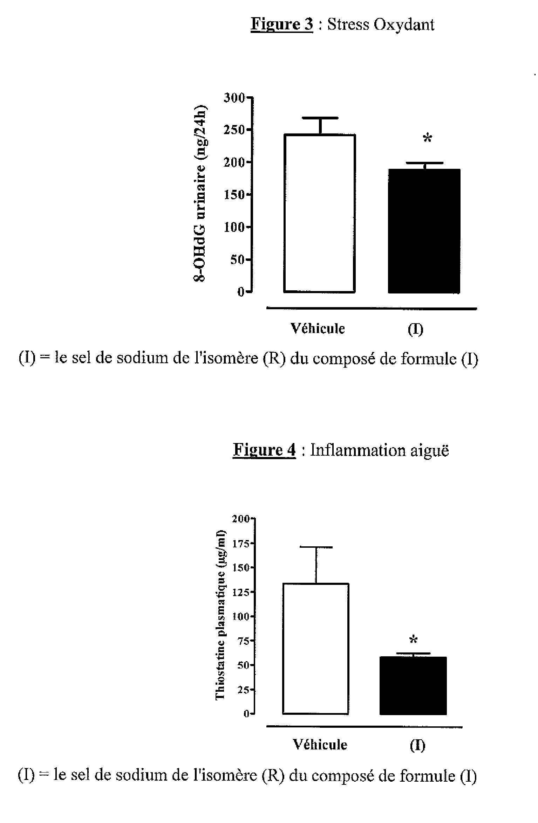

### 3) Stress Oxydant (Figure 3)

Les rats du groupe Vehicule présentent un stress oxydant qui peut-être évalué grâce à des marqueurs urinaires telle que la 8 OH-dG. La quantité totale la 8 OH-dG présente dans les urines pendant 24 heures est diminuée pour les animaux du groupe sel de sodium de l'isomère (R) du composé de formule (I) par rapport aux animaux du groupe Vehicule.

Ce résultat montre que le sel de sodium de l'isomère (R) du composé de formule (I) permet de diminuer le niveau de stress oxydant.

### 4) Inflammation aiguë (Figure 4)

La thiostatine est une protéine induite lors de phase aiguë d'inflammation. Cette protéine est largement augmentée dans le plasma des rats du groupe Vehicule. Lorsque les animaux sont traités avec le sel de sodium de l'isomère (R) du composé de formule (I) la quantité de thiostatine plasmatique est plus faible.

Ce résultat montre que le sel de sodium de l'isomère (R) du composé de formule (I) permet de ralentir l'installation de l'inflammation aiguë.

### Données viniques

L'activité antithrombotique ex vivo du sel de sodium de l'isomère (R) du composé de formule (I) a été également évalué chez des patients à risque important de survenue d'AVC ischémique et une supériorité du composé de formule (I) versus l'aspirine après 10 jours d'administration a été démontré sur les principaux paramètres du modèle de chambre de perfusion (surface totale et surface dense du thrombus, % d'adhésion). Dans cette étude, le sel de sodium de l'isomère (R) du composé de formule(I) a également un effet sur les marqueurs de l'inflammation mesurés (sVCAM, s PAI-1, sP-sélectine, ihrombomoduline).

L'étude de la réponse vasomotrice périphérique à une administration unique ou chronique du composé de formule (I) chez des patients athéromateux à risque cardiovasculaire important et test d'hyperhémie anormal traités par aspirine a mis en évidence une supériorité du composé de formule (I) versus placebo sur la vasodilatation dépendante de l'endothélium évaluée par le test d'hyperhémie provoquée (stimulus physiologique au niveau de l'artère radiale). Cette supériorité est observée quelle que soit la dose, après administration unique ou répétée (15 jours), l'effet vasodilatatoire étant évalué environ 2 heures après la prise médicamenteuse.

### Conclusion :

Le composé de formule (I) sous forme d'isomère (R) et préférentiellement sous forme de sel de sodium qui possède des propriétés antiagrégantes plaquettaires et anti-thrombotiques, anti-inflammatoires, anti-prolifératives et anti-vasoconstrictrices apparaît donc comme un agent anti-athérothrombotique pour une utilisation en prévention secondaire des accidents cardio-vasculaires dans les maladies athérothrombotiques, en particulier chez des patients avec antécédent d'accident ischémique cérébral.

## Revendications

1. Acide 3-{6-[(4-ehloro-phénylsulfonyl)amino]-2-méthyl-5,6,7,8-tétrahydronapht-1-yl}piopionique de formule (I) sous forme d'isomère optique de configuration (R) ou d'un de ses sels pharmaceutiquement acceptable pour son utilisation comme médicament dans la réduction des événements céréhro-vasculaires et cardio vasculaires chez des patients présentant un antécédent d'accident ischémique cérébral.

2. Acide 3-{6-[(4-chloro-phénylsulfonyl)amino]-2-méthyl-5,6,7,8-tétrahydronapht-1-yl}propionique de formule (I) sous forme d'isomère optique de configuration (R) ou d'un de ses sels pharmaceutiquement acceptable selon la revendication 1 pour son utilisation comme médicament dans la réduction des événements cérébro-vasculaires et cardio vasculaires chez des patients présentant un antécédent d'attaque ischémique transitoire.

3. Acide selon la revendication 1 ou 2 **caractérisée en ce que** l'isomère (R) du composé de formule (I) se présente sous la forme d'un sel de sodium.

4. Composition pharmaceutique comprenant l'acide 3-{6-[(4-chloro-phénylsulfonyl)amino]-2-méthyl-5,6,7,8-tétrahydronapht-1-yl}propionique de formule (I) sous forme d'isomère optique de configuration (R) ou d'un de ses sels pharmaceutiquement acceptable pour son utilisation comme médicament dans la réduction des événements cérébro-vasculaires et cardio vasculaires chez des patients présentant un antécédent d'accident ischémique cérébral.

5. Composition pharmaceutique selon la revendication 4 **caractérisée en ce que** l'acide 3-{6-[(4-chloro-phénylsulfonyl)amino]-2-méthyl-5,6,7,8-tétrahydronapht-1-yl}propionique de formule (I) de configuration (R) est sous forme de sel de sodium.

6. Composition pharmaceutique selon la revendication 5 **caractérisé en ce que** la quantité de sel de sodium de l'isomère (R) du composé de formule (I) est comprise entre 5 et 100 mg.

7. Composition pharmaceutique selon la revendication 5 ou 6 pour l'utilisation du sel de sodium de l'isomère (R) du composé de formule (I) à une dose journalière 30 mg par jour.
